(19) Europäisches Patentamt
European Patent Office
Office européen des brevets

(11) EP 1 339 375 B1

(12) # FASCICULE DE BREVET EUROPEEN

(45) Date de publication et mention de la délivrance du brevet:
**11.11.2009 Bulletin 2009/46**

(21) Numéro de dépôt: **01997282.7**

(22) Date de dépôt: **22.11.2001**

(51) Int Cl.:
***A61K 8/02** (2006.01)* ***A61Q 3/02** (2006.01)*

(86) Numéro de dépôt international:
**PCT/FR2001/003694**

(87) Numéro de publication internationale:
**WO 2002/041852 (30.05.2002 Gazette 2002/22)**

(54) **COMPOSITION COSMETIQUE A PHASE CONTINUE LIPOPHILE CONTENANT DES FIBRES**

KOSMETISCHE ZUSAMMENSETZUNG MIT EINER KONTINUIERLICHEN LIPOPHILEN PHASE, DIE FASERN ENTHÄLT

COSMETIC COMPOSITION WITH CONTINUOUS LIPOPHILIC PHASE CONTAINING FIBRES

(84) Etats contractants désignés:
**AT BE CH CY DE DK ES FI FR GB GR IE IT LI LU MC NL PT SE TR**

(30) Priorité: **23.11.2000 FR 0015134**

(43) Date de publication de la demande:
**03.09.2003 Bulletin 2003/36**

(73) Titulaire: **L'ORÉAL**
**75008 Paris (FR)**

(72) Inventeurs:
- **BLIN, Xavier**
  **F-75006 Paris (FR)**
- **JAGER LEZER, Nathalie**
  **F-91000 VERRIERES-LE-BUISSON (FR)**

(74) Mandataire: **Boulard, Denis**
**L'OREAL - D.I.P.I.**
**25-29 Quai Aulagnier**
**92600 Asnières (FR)**

(56) Documents cités:
**EP-A- 0 921 217 WO-A-97/38078**
**FR-A- 1 529 329 US-A- 6 001 338**

- **DATABASE CHEMICAL ABSTRACTS [en ligne] STN; access number 107: 223 060, XP002175565 & JP 62 164731 A (SEITETSU CHEM. IND. CO., LTD) 21 juillet 1987 (1987-07-21)**
- **Traduction en Anglais de JP 62 164 731 (SEITETSU), d'où est extrait l'abrégé de Chemical abstracts précédemment cité..**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 339 375 B1

Printed by Jouve, 75001 PARIS (FR)

## Description

La présente invention se rapporte à une composition cosmétique contenant des fibres plates, et plus spécialement à une composition de maquillage de la peau aussi bien du visage que du corps humain, des lèvres et des phanères comme les onglets, les cils, les sourcils ou les cheveux.

La composition selon l'invention peut être une composition de maquillage comme les produits pour le teint (fonds de teint colorés ou non), les fards à joues ou à paupières, les produits pour les lèvres colorés ou non, les produits anti-cernes, les blush, les mascaras colorés ou non, les eye-liners, les produits de maquillage des sourcils, les crayons à lèvres ou à yeux, les produits pour les ongles colorés ou non, les produits de maquillage du corps, les produits de maquillage des cheveux (mascara ou laque pour cheveux), ou bien encore une composition pour le soin de la peau comme les compositions destinées au camouflage des imperfections de la peau. La composition peut être utilisée telle quelle pour l'application sur les matières kératiniques ou bien encore être appliquée sur un maquillage déjà déposé sur les matières kératiniques, par exemple pour modifier le maquillage (la composition est appliquée comme produit de surface communément appelée top coat en terminologie anglo-saxonne).

La composition de maquillage peut également être appliquée sur les accessoires de maquillage (support) comme les faux ongles, faux cils, postiches, perruques ou encore sur des pastilles ou patchs adhérents sur la peau ou les lèvres (du type mouches).

Il est connu d'utiliser des fibres dans des produits de maquillage notamment pour leurs effets allongeant dans des mascaras (voir JP-A-57/158714), leurs propriétés hydratantes dans des rouges à lèvres (voir le document US-A-5 498407), pour améliorer les contours du rouge à lèvres sur les bords des lèvres (voir le document EP-A-0106762) ou pour remettre en état les ongles cassés (voir FR-A-1529329) ou bien encore dans des produits de soin de la peau pour leur toucher velouté (voir JP-A-7/196440). La demande JP2000-319131 décrit des compositions à base de poudres polymériques rectangulaires. Malheureusement, il est très difficile de disperser des fibres dans des compositions, de façon homogène et sans former d'amas, ce qui dans une composition cosmétique, et en particulier dans une composition de maquillage, confère généralement un dépôt sur les matières kératiniques, notamment un maquillage, non uniforme et peu esthétique, à contour peu net. De plus, les amas de fibres dûs à la mauvaise dispersion des fibres modifient l'aspect visuel de la composition.

En outre, cette difficulté de dispersion conduit à des compositions présentant des propriétés cosmétiques non constantes et peu reproductibles, et entraîne également des problèmes de fabrication industrielle et des coûts élevés de fabrication.

La but de la présente invention est donc de proposer une composition topique ne présentant pas les inconvénients ci-dessus et comprenant des fibres réparties de façon homogène.

Les inventeurs ont découvert que les fibres plates, c'est à dire des fibres dont la section présente une forme aplatie, s'incorporent très facilement dans les compositions cosmétiques et se répartissent dans la composition de façon homogène.

L'incorporation des fibres plates dans la composition se fait très facilement, aussi bien à froid qu'à chaud sans perdre les propriétés cosmétiques de la composition. En particulier, la bonne homogénéité des fibres dans la composition ne change pas l'aspect du produit. Il est possible d'incorporer des teneurs importantes en fibres plates dans la composition sans modifier l'aspect visuel de la composition.

La composition appliquée sur les matières kératiniques forme un dépôt présentant une sensation de velours au toucher dû à la dispersion homogène des fibres plates dans la composition et dans le dépôt formé après l'application. La composition apporte donc un toucher différent des touchers lisses, craquelés ou granuleux, satisfaisant ainsi les consommateurs à la recherche de nouveauté.

En outre, les fibres plates apportent des propriétés de renfort mécanique de la composition et du dépôt formé après application sur les matières kératiniques, en particulier lorsque ie dépôt comprend un polymère filmogène. En particulier, la composition forme un dépôt présentant de bonnes propriétés de résistance mécanique : le dépôt est bien résistant aux frottements, aux chocs, aux rayures. Le dépôt est également bien résistant à l'eau (notamment lors de la baignade ou de la douche), de la pluie, des larmes, de la sueur, du sébum. Les fibres plates apportent ainsi une meilleure tenue du dépôt sur les matières kératiniques.

La composition comprenant des fibres plates appliquée sur la peau permet d'obtenir un bon camouflage des imperfections de la peau.

Les fibres plates confèrent également un effet visuel brillant. Lorsqu'elles sont formulées dans un support transparent ou translucide, la composition peut être appliquée comme top coat sur un maquillage déjà déposé sur les matières kératiniques pour ainsi modifier l'aspect du maquillage.

En outre, l'effet de couleur particulier du maquillage est bien visible lorsque la composition est appliquée sur des peaux sombres comme les peaux éthniques.

De façon plus précise, l'invention a pour objet une composition cosmétique à application topique à phase continue lipophile, et plus spécialement une composition cosmétique de maquillage à phase continue lipophile, contenant des fibres plates plus précisément décrites dans la présente revendication 1.

L'invention a aussi pour objet un procédé cosmétique de maquillage ou de soin des matières kératiniques comprenant l'application sur les matières kératiniques d'une composition telle que définie précédemment.

L'invention a encore pour objet l'utilisation de fibres plates selon la revendication 1 dans une composition cosmétique comprenant une phase continue lipophile pour renforcer mécaniquement le dépôt obtenu après l'application de la dite composition sur les matières kératiniques, et/ou ladite composition.

L'invention a aussi pour objet un procédé cosmétique de maquillage des matières kératiniques comprenant l'application sur les matières kératiniques d'une première couche, appelée aussi couche de base, d'une première composition cosmétique comprenant dans un milieu cosmétiquement acceptable au moins une matière colorante, puis l'application sur au moins une partie de ladite première couche, d'une deuxième couche d'une deuxième composition cosmétique comprenant, dans un milieu continu lipophile cosmétiquement acceptable, des fibres plates selon la revendication 1, la première composition ne comprenant pas de fibres plates comme présentes dans la deuxième composition.

L'invention a également pour objet un kit de maquillage comprenant :

- une première composition comprenant au moins une matière colorante dans un milieu cosmétiquement acceptable, et
- une deuxième composition comprenant des fibres plates selon la revendication 1 dans un milieu continu lipophile cosmétiquement acceptable,

la première composition ne comprenant pas de fibres plates comme présent dans la deuxième composition,
les première et deuxième compositions étant conditionnées dans des récipients distincts.

L'invention a également pour objet un support maquillé, tels que les accessoires de maquillage cités précédemment, comprenant un maquillage susceptible d'être obtenu selon le procédé de maquillage tel que défini précédemment et appliqué sur ledit support.

Par "fibre", il faut comprendre un objet de longueur L et de diamètre D tel que L soit très supérieur à D, D étant le diamètre du cercle dans lequel s'inscrit la section (section transversale) de la fibre. En particulier, le rapport L/D (ou facteur de forme) est choisi dans la gamme allant de 1,2 à 2 500, de préférence de 1,5 à 500, et mieux de 1,6 à 150.

Par "fibre plate", on entend une fibre dont la section transversale (section perpendiculaire à l'axe de la direction de la longueur de la fibre) présente une plus grande longueur L1 et une plus petite longueur L2 (L2 correspond à l'épaisseur de ia fibre) telle que L1/L2 (le rapport L1/L2 est encore appelé facteur d'aplatissement) est supérieur ou égal à 4, de préférence supérieur à 7. Notamment, L1/L2 va de 4 à 15, de préférence de 6 à 12 , et mieux de 7 à 10. Ainsi, la section transversale de la fibre présente une forme plate. Avantageusement, la plus grande longueur L1 et la plus petite longueur L2 définissent respectivement des axes X1, X2 tels que l'axe X1 est sensiblement perpendiculaire à l'axe X2. La plus grande longueur L1 correspond au diamètre D de la fibre tel que mentionné précédemment. Ainsi, les fibres plates peuvent se présenter sous la forme de ruban ou de tagliatelle.

Les fibres plates présentent une section transversale de forme ovoïdale ou ellipsoïdale.

Les fibres utilisables dans la composition de l'invention sont des fibres d'origine synthétique, organique, et plus particulièrement des fibres de polymère synthétique. Elles peuvent être courtes ou longues, unitaires (ou monofilament) ou organisées par exemple tressées (ou multi-filaments), creuses ou pleines, de préférence pleine. Lorsque les fibres sont des fibres multifilaments, chaque filament peut être de composition chimique différente et présenter une couleur différente : on obtient ainsi des fibres multifilaments présentant des couleurs différentes. En particulier, leurs extrémités sont épointées et/ou polies pour éviter de se blesser.
Avantageusement, les fibres plates sont insolubles dans l'eau.
La fibre plate peut être torsadée le long de l'axe de la longueur L de la fibre. Lorsque la fibre plate n'est pas torsadée, elle présente une couleur dans un certain angle de vue, en dehors de cet angle la fibre est transparente ou de couleur blanche. La fibre plate torsadée quant à elle présente une couleur quel que soit l'angle d'observation.

En particulier, les fibres ont une longueur allant de 1 μm à 10 mm, de préférence de 0,1 mm à 5 mm et mieux de 0,3 mm à 3,5 mm. Leur section transversale (section plate) peut être comprise dans un cercle de diamètre allant de 2 nm à 500 μm, de préférence allant de 100 nm à 100 μm et mieux de 1 μm à 70 μm. Le poids ou titre des fibres est souvent donné en denier ou décitex et représente le poids en gramme pour 9 km de fil. De préférence, les fibres selon l'invention ont un titre choisi dans ia gamme allant de 0,15 à 30 deniers et mieux de 0,18 à 18 deniers.

Les fibres peuvent être à base de rayonne, de polyamide (Nylon®), de viscose, d'acétate notamment d'acétate de rayonne, de poly-(p-phénylène-téréphtalamide) (ou d'aramide) notamment de Kevlar®, de polymère acrylique notamment de polyméthacrylate de méthyle ou de polyméthacrylate de 2-hydroxyéthyle, de polyoléfine et notamment de polyéthylène ou de polypropylène, de polytétrafluoroéthylène (comme le Téflon®), de polychlorure de vinyle ou de vinylidène, de polyfluorure de vinylidène, d'alcool polyvinylique, de polyacrylonitrile, de polyuréthane, de polyesters comme les polyéthylène téréphtalates, les polyéthylène naphtalates, de polycarbonate.

Par ailleurs, les fibres peuvent être traitées ou non en surface, enrobées d'une couche de protection ou non.

Les fibres utilisables dans la composition selon l'invention sont préférentiellement des fibres de polyester, de

polymère acrylique, de polyamide.

Les fibres plates sont des fibres à structure multicouche de polymère, lesdites couches étant telles qu'elles permettent la création d'un effet de couleur par interférences des rayons lumineux, qui diffractent et diffusent différemment selon les couches. Ainsi de telles fibres peuvent présenter des couleurs variant selon l'angle d'observation et l'incidence de la lumière, et peuvent conférer des reflets irisés. Des fibres à structure multicouche de polymères sont notamment décrites dans les documents EP-A-921217, EP-A-686858 et US-A-5472798.

La structure multicouche comporte au moins deux couches, chaque couche, indépendamment ou non de la (ou les) autre(s) couche(s), étant réalisée en au moins un polymère de synthèse.

La fibre plate est formée de couches individuelles alternées de polymères ayant des indices de réfraction différents ; chaque couche étant dans un plan (P) parallèle à la direction de l'axe principal de la fibre, dans le sens de sa longueur L. Selon l'épaisseur de chacune des différentes couches, on obtient différentes couleurs. En général, la structure est composée d'une alternance de couches de bas indice de réfraction et de haut indice de réfraction. Ainsi, en coupe transversale à la direction de l'axe de la longueur L de la fibre, la fibre a une structure multicouche comprenant des couches alternées d'au moins un premier polymère et un deuxième polymère.

La partie multicouche de la fibre peut comprendre au moins 5 couches individuelles de polymère, notamment de 5 à 120, de préférence au moins 10 couches, notamment de 10 à 70 couches, et mieux de 10 à 50 couches.

Chaque couche des premier et deuxième polymères a respectivement une épaisseur $d_1$, $d_2$ qui peut aller, indépendamment l'une de l'autre, de 0,02 µm à 0,3 µm, et de préférence de 0,05 µm à 0,15 µm.

Avantageusement, les polymères présents dans les fibres ont avantageusement un indice de réfraction allant de 1,30 à 1,82 et mieux allant de 1,35 à 1,75. En particulier, les premier et deuxième polymères ont respectivement un indice de réfraction $n_1$ et $n_2$ tels que $n_1/n_2$ va de 1,1 à 1,4.

Avantageusement, $n_1$, $n_2$, $d_1$, $d_2$ satisfont à l'équation :

$$\lambda = 2(n_1d_1 + n_2d_2) = 2\ n_1[d_1 + d_2(n_2/n_1)]$$

dans laquelle $\lambda$ est la longueur d'onde, exprimée en µm, de la couleur de la fibre formée par interférence optique (longueur d'onde du pic du spectre de réflexion) ; $d_1$ et $d_2$ étant exprimés en µm.

La fibre plate à structure multicouche présente de préférence un spectre de réflexion tel que la largeur à mi-hauteur du spectre $\lambda_{L=1/2}$ est dans la gamme $0 < \lambda_{L=1/2} < 200$ nm.

Les polymères constituant les fibres particulièrement préférés sont les polyesters tels que le polyéthylène téréphtalate, le polyéthylène naphtalate, le polycarbonate ; les polymères acryliques comme le polyméthacrylate de méthyle ; les polyamides.

Le polyéthylène téréphtalate peut être obtenu par polycondensation d'acide téréphtalique et d'éthylèneglycol. Il peut contenir de 0 à 30 % en poids, de préférence de 0 à 15 % en poids, et mieux de 0 à 10 % en poids, par rapport au poids total de monomères, d'autres comonomères.

En particulier, le polyéthylène téréphtalate peut comprendre de 0,3 à 10 % en mole, et de préférence de 0,5 % à 5 % en mole, de monomère diacide carboxylique comportant un groupe acide sulfonique neutralisé par un sel métallique, par rapport au poids total de monomère diacide carboxylique présent dans le polyéthylène téréphtalate.

Le groupe acide sulfonique neutralisé par un sel de métallique est un groupe de formule $-SO_3M$ dans lequel M est un métal, de préférence un métal alcalin ou alcalino-terreux, et plus particulièrement le sodium, le potassium ou le lithium.

Comme exemple de monomère diacide carboxylique comportant un groupe acide sulfonique neutralisé par un sel métallique, on peut utiliser le sel de sodium de l'acide 5-sulfoisophtalique, le sel de potassium de l'acide 5-sulfoisophtalique, le sel de carbonyl)benzènesulfonate de potassium, le 3,5-di(β-hydroxyéthoxycarbonyl) benzènesulfonate de lithium, le diester méthylique de l'acide 4-sulfonate-2,6-naphtalique de sodium, le diester méthylique de l'acide 4-sulfonate-2,6-naphtalique de potassium, le diester méthylique de l'acide 4-sulfonate-2,6-naphtalique de lithium, le 2,6-dicarboxynaphtalène-4-sulfonate de sodium, le 2,6-dicarboxynaphtalène-1-sulfonate de sodium, le diester méthylique de l'acide 3-sulfonate-2,6-naphtalique, le diester méthylique de l'acide 4,8-disulfonate-2,6-naphtalique de sodium, le 2,6-dicarboxynaphtalène-4,8-disulfonate de sodium, le 2,5-bis(hydroxyéthoxy) benzènesulfonate de sodium, le sulfosuccinate de sodium, et leurs mélanges. On utilise de préférence le diester méthylique de l'acide 5-sulfoisophtalique de sodium, le sel de sodium de l'acide 5-sulfoisophtalique et le 3,5-di(β-hydroxyéthoxycarbonyl) benzènesulfonate de sodium.

Le polyéthylène naphtalate peut être obtenu par polycondensation d'acide 2-6-naphtalique ou d'acide 2,7-naphtalique et d'éthylène glycol. Le polyéthylène naphtalate peut donc être un polyéthylène-2,6-naphtalate ou un polyéthylène-2,7-naphtalate, de préférence un polyéthylène-2,6-naphtalate.

Il peut contenir de 0,3 % à 5 % en mole de monomère diacide carboxylique comportant un groupe acide sulfonique neutralisé par un sel métallique tel que défini précédemment, par rapport au poids total de monomère diacide carboxylique présent dans le poléthylène naphtalate.

D'autres comonomères tels qu'un diacide carboxylique additionnel, différents des diacides carboxyliques mentionnés précédemment, ou un diol additionnel, différent de polyéthylène glycol, peuvent être présents dans le polyéthylène téréphtalate ou le polyéthylène naphtalate.

Le diacide carboxylique additionnel peut être choisi parmi les diacides carboxyliques aromatiques tels que l'acide isophtalique, l'acide biphényl dicarboxylique, l'acide 4,4'-dicarboxylique de diphényléther, l'acide 4,4'-dicarboxylique de diphénylméthane, l'acide 4,4'-dicarboxylique de diphénylsulfone, l'acide 4,4'-dicarboxylique de 1,2-diphénoxyéthane, l'acide 2,5-dicarboxylique de pyridine, l'acide 2,6-dicarboxylique naphtalène, l'acide 2,7-dicarboxylique naphtalène, le diacide carboxylique de diphénylcétone ; les diacides carboxyliques aliphatiques tels que l'acide malonique, l'acide succinique, l'acide adipique, l'acide azéiaïque, l'acide sébacique ; ies acides dicarboxyliques alicycliques tels que le diacide carboxylique de décaline ; les acides hydroxycarboxyliques tels que l'acide β-hydroxyéthoxybenzoïque, l'acide parahydroxybenzoïque et l'acide hydroxypropionique.

Le diol additionnel peut être notamment choisi parmi les diols aliphatiques tels que le propylèneglycol, le butylèneglycol, l'hexylèneglycol, le diéthylèneglycol, le polyéthylèneglycol ; les diols aromatiques tels que l'hydroquinone, le catéchol, le naphtalènediol, la résorcine, le bisphénol A ; les diols alicycliques tels que le cyclohexanediméthanol.

D'autres comonomères peuvent être également choisis parmi les acides carboxyliques polyvalents tels que l'acide trimellitique, l'acide pyromellitique, l'acide tricarballylique ; les alcools polyhydriques tels que la glycérine, le triméthyloléthane, le triméthylolpropane et le pentaérythritol.

Le polyméthylméthacrylate peut comprendre des monomères acides tels que l'indice d'acide du polymère soit de préférence supérieur à 3, notamment allant de 3 à 20, et mieux de 4 à 15. De tels monomères acides peuvent être l'acide (méth)acrylique ou l'acide maléique.

Le polyamide peut être choisi parmi le nylon 6, nylon 6-6, nylon 6-12, nylon 11, nylon 12, dont la composition chimique est bien connue de l'homme du métier.

Avantageusement, dans la structure multicouche des fibres plates, le premier polymère peut être choisi parmi les polyesters tels que le polyéthylène téréphtalate, le polyéthylène naphtalate, le polycarbonate, notamment ceux définis précédemment ; le deuxième polymère peut être choisi parmi les polymères acryliques comme le polyméthacrylate de méthyle, et les polyamides, notamment ceux décrits précédemment.

Les fibres plates à structure multicouche peuvent comporter une couche de protection qui peut comprendre un polymère choisi parmi les polymères de couche. De préférence, le polymère de la couche de protection peut avoir un indice de réfraction allant de 1,35 à 1,55.
L'épaisseur de la couche de protection peut être plus grande que l'épaisseur des couches de polymères de la partie multicouche.
L'épaisseur de la couche de protection peut aller de 2 μm à 10 μm, et de préférence de 2 μm à 7 μm.

Lorsque la fibre comporte en surface une couche de protection, cette couche de protection est prise en compte pour le calcul du facteur d'aplatissement.

Comme polymère de la couche de protection, on peut notamment utiliser le polytétrafluoroéthylène, les copolymères tétrafluoroéthylène/propylène, les copolymères tétrafluoroéthylène/hexafluoropropylène, les copolymères tétrafluoroéthylène/éthylène, les copolymères tétrafluoroéthylène / tétrafluoro propylène, le polyfluorure de vinylidène, les polyacrylates de pentadécafluorooctyl, les polyacrylates de fluoroéthyle, les polyméthacrylates de trifluoroisopropyle, les polyméthacrylates de trifluoroéthyle, les polyacrylates d'éthyle, les polyméthacrylates d'éthyle. On peut également utiliser des polymères siliconés tels que les polydiméthylsilanes, les polydiméthylsiloxanes ; des polyuréthanes.

Les fibres plates peuvent être obtenues de façon connue par extrusion du ou des polymères à travers une filière de forme rectangulaire puis découpage du fil obtenu à la longueur voulue.

Comme fibres plates, on peut utiliser les fibres interférentielles vendues sous les dénominations "Morphotex" ou « Teijin Tetron Morphotex » par la société TEIJIN. De telles fibres plates sont décrites dans la demande EP-A-921217.

Les fibres plates peuvent êtres présentes dans la composition selon l'invention, notamment dans la composition de surface, en une teneur allant de 0,01 % à 50 % en poids, par rapport au poids total de la composition, de préférence de 0,1 % à 30 % en poids, et mieux de 0,3 % à 20 % en poids.

La composition de l'invention contenant les fibres plates peut se présenter sous forme d'un produit à appliquer sur la peau, les lèvres, et/ou les phanères d'êtres humains. Elle contient donc un milieu cosmétiquement acceptable, c'est-à-dire un milieu compatible avec toutes les matières kératiniques telles que la peau aussi bien du corps humain que du visage, les ongles, les cheveux, les cils et sourcils.

Selon l'invention, ce milieu contient une phase continue lipophile, c'est-à-dire un mélange d'un ou plusieurs corps gras ou de solvants organiques, non miscibles à l'eau ou miscible à moins de 50 % en poids dans l'eau à température ambiante (25 °C), qui peuvent être liquides, pâteux ou solides à température ambiante (25°C en général). En particulier, ce milieu peut comprendre ou se présenter notamment sous forme de suspension, dispersion ou solution dans une

phase huileuse ou de solvant organique lipophile, éventuellement épaissie, voire gélifiée ; suspension ou dispersion dans une phase cireuse ; émulsion eau-dans-huile (E/H), ou multiple (H/E/H) sous forme de crème ou de pâte ; gel anhydre ou mousse huileux ; gel émulsionné ; lotion biphase ou multiphase ; spray ; poudre ; pâte anhydre. L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition comprend donc une phase continue lipophile qui peut contenir des corps gras liquides à température ambiante et pression atmosphérique, appelés souvent huiles, des solvants organiques non miscibles à l'eau, des cires, des gommes, des corps gras pâteux ou un mélange de ces constituants. Cette phase continue peut représenter de 0,5 à 99,99% du poids total de la composition.

Dans le kit selon l'invention, la première composition, dite composition de base, peut comprendre comme milieu cosmétiquement acceptable un milieu aqueux ou un milieu solvant organique.

Comme corps gras liquides à température ambiante, appelés souvent huiles, utilisables dans l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras ou d'alcool gras ayant de 8 à 26 atomes de carbone, comme par exemple l'huile de Purcellin, le myristate d'isopropyle, le palmitate d'éthyl-2hexyle, le stéarate d'octyl-2-dodécyle, l'érucate d'octyl-2-dodécyle, l'isostéarate d'isostéaryle ; les esters d'acides gras ou d'alcool gras hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes leurs mélanges.

Ces huiles peuvent représenter de 0 à 99,99 % en poids par rapport au poids total de la phase grasse.

La phase continue lipophile de la composition selon l'invention peut également comprendre un ou plusieurs solvants organiques, cosmétiquement acceptables (tolérance, toxicologie et toucher acceptables). Ces solvants organiques peuvent représenter de 0 à 90 % du poids total de la composition et peuvent être choisis dans le groupe constitué par les solvants organiques lipophiles, les solvants amphiphiles ou leurs mélanges. Comme solvants organiques utilisables dans la composition de l'invention, on peut citer les esters de l'acide acétique comme l'acétate de méthyle, d'éthyle, de butyle, d'amyle, de méthoxy-2-éthyle, l'acétate d'isopropyle; les cétones comme la méthyléthylcétone, la méthylisobutylcétone ; les hydrocarbures comme le toluène, le xylène, l'hexane, l'heptane ; les aldéhydes ayant de 5 à 10 atomes de carbone ; les éthers ayant au moins 3 atomes de carbones et leurs mélanges.

Lorsque la composition selon l'invention, ou l'une des compositions de base et/ou de surface, se présente sous la forme d'une émulsion, elle peut éventuellement comprendre, en outre, un tensioactif, de préférence en une quantité de 0 à 30% et notamment de 0,01 à 30% en poids par rapport au poids total de la composition.

Selon l'application envisagée, la composition selon l'invention, et notamment la première et/ou la deuxième composition, peut comprendre, en outre, un polymère filmogène. Le polymère filmogène peut être un polymère solubilisé ou dispersé sous forme de particules dans la phase continue lipophile de la composition ; il peut également être solubilisé ou dispersé dans une phase aqueuse. La composition peut comprendre un mélange de ces polymères.

Le polymère filmogène peut être présent dans la composition selon l'invention, ou l'une des compositions de base et/ou de surface, en une teneur en matières sèches de polymère allant de 0,1 % à 60 % en poids par rapport au poids total de la composition, de préférence de 0,5 % à 40 % en poids, et mieux de 1 % à 30 % en poids.

Dans la présente demande, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique chaque monomère étant susceptible de s'homopolymériser (à l'inverse des polycondensats).
Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$ .

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de iauryie, ie méthacrylate de cyclohexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol.

Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement $-SO_3M$, avec M représentant un atome d'hydrogène, un ion ammonium $NH_4^+$ ou un ion métallique, comme par exemple un ion $Na^+$, $Li^+$, K+, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$.

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$ : l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ® par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques insolubles dans l'eau tels que la nitrocellulose, l'acétate de cellulose, l'acétobutyrate de cellulose, l'acétopropionate de cellulose, l'éthyl cellulose, et leurs mélanges.

Selon un premier mode de réalisation de la composition selon l'invention, le polymère filmogène peut être présent sous la forme de particules, stabilisées en surface, dispersées dans la phase grasse liquide. Les particules de polymère filmogène peuvent avoir une taille allant de 5 nm à 600 nm, et notamment allant de 20 nm à 300 nm. Les techniques de préparation de ces dispersions sont bien connues de l'homme du métier. Des dispersions de polymère filmogène dans la phase grasse liquide, en présence d'agents stabilisants, sont notamment décrites dans les documents EP-A-749746, EP-A-923928, EP-A-930060.

Selon une deuxième variante de réalisation de la composition selon l'invention, le polymère filmogène peut être solubilisé dans la phase grasse liquide, on dit alors que le polymère filmogène est un polymère liposoluble.

A titre d'exemple de polymère liposoluble, on peut citer les polymères correspondant à la formule (I) suivante :

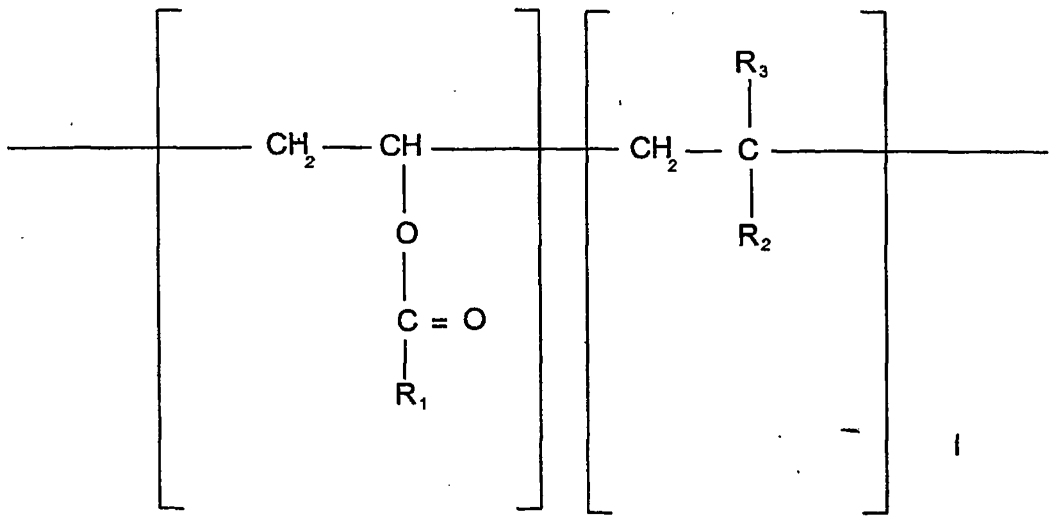

I

Ia Ib

dans laquelle :

- $R_1$ représente une chaîne hydrocarbonée saturée, linéaire ou ramifiée, ayant de 1 à

  a) $-O-CO-R_4$, $R_4$ ayant la même signification que $R_1$ mais est différent de $R_1$ dans un même copolymère,

  b) $-CH_2-R_5$, $R_5$ représentant une chaîne hydrocarbonée saturée, linéaire ou ramifiée, ayant de 5 à 25 atomes de carbone,

c) $-O-R_6$, $R_6$ représentant une chaîne hydrocarbonée saturée, ayant de 2 à 18 atomes de carbone, et

d) $-CH_2-O-CO-R_7$, $R_7$ représentant une chaîne hydrocarbonée saturée, linéaire ou ramifiée, ayant de 1 à 19 atomes de carbone,

- $R_3$ représente un atome d'hydrogène quand $R_2$ représente les radicaux a), b) ou c) ou $R_3$ représente un radical méthyle quand $R_2$ représente le radical d), ledit copolymère devant être constitué d'au moins 15 % en poids d'au moins un monomère dérivé d'un motif (Ia) ou d'un motif (Ib) dans lesquels les chaînes hydrocarbonées saturées, linéaires ou ramifiées, ont au moins 7 atomes de carbone.

Les copolymères de formule (I) résultent de la copolymérisation d'au moins un ester vinylique (correspondant au motif Ia) et d'au moins un autre monomère (correspondant au motif Ib) qui peut être une $\alpha$-oléfine, un alkylvinyléther ou un ester allylique ou méthalylique.

Lorsque dans le motif (Ib) $R_2$ est choisi parmi les radicaux $-CH_2-R_5$, $-O-R_6$ ou $-CH_2-O-CO-R_7$ tels que définis précédemment, le copolymère de formule (I) peut être constitué de 50 à 95 % en moles d'au moins un motif (Ia) et de 5 à 50 % en moles d'au moins un motif (Ib).

Les copolymères de formule (I) peuvent également résulter de la copolymérisation d'au moins un ester vinylique et d'au moins un autre ester vinylique différent du premier. Dans ce cas, ces copolymères peuvent être constitués de 10 à 90 % en moles d'au moins un motif (Ia) et de 10 à 90% en moles d'au moins un motif (Ib) dans lequel $R_2$ représente le radical $-O-CO-R_4$.

Parmi les esters vinyliques conduisant au motif de formule (Ia), ou au motif de formule (Ib) dans lequel $R_2 = -O-CO-R_4$, on peut citer l'acétate de vinyle, le propionate de vinyle, le butanoate de vinyle, l'octanoate de vinyle, le décanoate de vinyle, le laurate de vinyle, le stéarate de vinyle, l'isostéarate de vinyle, le diméthyl-2, 2 octanoate de vinyle, et le diméthylpropionate de vinyle.

Parmi les $\alpha$-oléfines conduisant au motif de formule (Ib) dans lequel $R_2 = -CH_2-R_5$, on peut citer l'octène-1, le dodécène-1, l'octadécène-1, l'eicosène-1, et les mélanges d' $\alpha$-oléfines ayant de 22 à 28 atomes de carbone.

Parmi les alkylvinyléthers conduisant au motif de formule (Ib) dans lequel $R_2 = -O-R_6$, on peut citer l'éthylvinyléther, le n-butylvinyléther, l'isobutylvinyléther, le décylvinyléther, le dodécylvinyléther, le cétylvinyléther et l'octadécylvinyléther.

Parmi les esters allyliques ou méthallyliques conduisant au motif de formule (Ib) dans lequel $R_2 = -CH_2-O-CO-R_7$, on peut citer les acétates, les propionates, les diméthylpropionates, les butyrates, les hexanoates, les octanoates, les décanoates, les laurates, les diméthyl-2, 2 pentanoates, les stéarates et les eicosanoates d'allyle et de méthallyle.

Les copolymères de formule (I) peuvent également être réticulés à l'aide de certains types de réticulants qui ont pour but d'augmenter sensiblement leur poids moléculaire.

Cette réticulation est effectuée lors de la copolymérisation et les réticulants peuvent être soit du type vinylique, soit du type allylique ou méthallylique. Parmi ceux-ci, on peut citer en particulier le tétraallyloxyéthane, le divinylbenzène, l'octanedioate de divinyle, le dodécanedioate de divinyle, et l'octadécanedioate de divinyle.

Parmi les différents copolymères de formule (I) utilisables dans la composition selon l'invention, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 10 à 20 atomes de carbone.

De tels homopolymères liposolubles peuvent être choisis parmi le polystéarate de vinyle, le polystéarate de vinyle réticulé à l'aide de divinylbenzène, de diallyléther ou de phtalate de diallyle, le poly(méth)acrylate de stéaryle, le polylaurate de vinyle, le poly(méth)acrylate de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en $C_2$-$C_{20}$, différents de la cire de polyoléfine définie en a), comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Le polymère filmogène est en particulier utilisé lorsqu'on souhaite préparer une composition de type vernis à ongles, mascara, eye-liner, laque à sourcils ou composition capillaire notamment de coiffage.

Le polymère filmogène peut être associé à des agents auxiliaire de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition selon l'invention, ou l'une des compositions de base et/ou de surface, peut également comprendre une phase aqueuse, alcoolique ou hydroalcoolique, sous forme dispersée ou émulsionnée dans la phase continue. Cette phase peut contenir de l'eau, des alcools, un mélange d'eau et d'alcool ou d'acétone. Les alcools sont notamment .des monoalcools inférieurs linéaires ou ramifiés ayant de 2 à 5 atomes de carbone comme l'éthanol ou le propanol, des polyols comme la glycérine, la diglycérine, le propylène glycol, le sorbitol, le penthénol, le penthylène glycol, les polyéthylène glycols. Cette phase aqueuse peut représenter de 0 à 90% du poids de la composition. Elle peut, en outre, contenir des éthers en $C_2$ et des aldéhydes en $C_2$-$C_4$.

La composition selon l'invention, ou l'une des compositions de base et/ou de surface, peut, de plus, comprendre tous les ingrédients classiquement utilisés dans les domaines concernés et plus spécialement dans les domaines cosmétique et dermatologique. Ces ingrédients sont en particulier choisis parmi les conservateurs, les épaississants, les parfums, les actifs hydrophiles ou lipophiles et leurs mélanges. Les quantités de ces différents ingrédients sont celles classiquement utilisées dans les domaines concernés et par exemple de 0,01 % à 20 % du poids total de la composition.

La composition de l'invention, ou la composition de surface, peut, en outre, comprendre une phase particulaire additionnelle pouvant être présente à raison de 0 à 48 % (notamment 0,01 % à 48 %) du poids total de la composition, de préférence de 0,01 % à 30 % et mieux de 0,02 % à 20 %, et qui peut comprendre des pigments et/ou des nacres et/ou des charges utilisés dans les compositions cosmétiques.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0 à 25 % (notamment 0,01 % à 25 %) par rapport au poids de la composition, de préférence de 0,01 % à 15 % en poids, et mieux de 0,02 % à 5 % en poids. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium ou encore les dicéto pyrrolopyrrole (DPP) décrits dans les documents EP-A-542669, EP-A-787730, EP-A-787731 et WO-A- 96/08537.

Les nacres peuvent être présentes dans la composition à raison de 0 à 25 % (notamment 0,01 % à 25 %) en poids, par rapport au poids total de la composition, de préférence de 0,01 % à 15 % en poids, et mieux de 0,02 % à 5 % en poids. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 48 % en poids, par rapport au poids total de la composition, de préférence 0,01 à 30 % en poids, et mieux de 0,02 % à 20 % en poids. On peut notamment citer le talc, le stéarate de zinc, le mica, le kaolin, les poudres de polyamide (Nylon®) (Orgasol® de chez Atochem), les poudres de polyéthylène, les poudres de polymères de tétrafluoroéthylène (Téflon®), l'amidon, le nitrure de bore, des microsphères polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les organopolysiloxanes élastomères.

La composition peut comprendre également des colorants hydrosolubles ou liposolubles en une teneur allant de 0 à 6 % (notamment 0,01 % à 6 %) en poids, par rapport au poids total de la composition, de préférence allant de 0,01 % à 3 % en poids. Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine. Les colorants hydrosolubles sont par exemple le jus de betterave, le bleu de méthylène.

Selon un mode préféré du kit et du procédé de maquillage selon l'invention, la composition de base est colorée par la matière colorante et la composition de surface peut comprendre un milieu sensiblement translucide de telle sorte

que ledit milieu ne masque pas la couleur des fibres plates. L'application de la composition de base sur les matières kératiniques puis de la composition de surface conduit alors à un maquillage faisant apparaître de manière distincte les fibres plates, réparties sur la couche colorée obtenue avec la composition de base. On observe alors un contraste entre les couleurs des fibres plates et la couleur de la couche de base.

La matière colorante présente dans la première composition de base du kit et du procédé de maquillage peut être choisie parmi les pigments, les nacres et les colorants hydrosolubles ou liposolubles tels que définis précédemment, selon les teneurs indiquées.

La composition de l'invention, ou l'une des compositions de base et/ou de surface, peut comprendre une ou plusieurs gommes et/ ou une ou plusieurs cires. Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des PDMS à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition, ou l'une des compositions de base et/ou de surface, peut contenir de 0 à 50 % en poids de cires, par rapport au poids total de la composition et mieux de 1 à 30 %.

Cette composition peut avoir l'aspect d'une crème, pommade, lotion fluide, de pâte souple (notamment de pâte ayant de viscosité dynamique à 25°C de l'ordre de 0,1 à 40 Pa.s sous une vitesse de cisaillement de 200 $s^{-1}$, après 10 minutes de mesure en géométrie cône/plan), onguent.

La composition selon l'invention, ou l'une des compositions de base et/ou de surface, peut également contenir des ingrédients couramment utilisés en cosmétique, tels que les vitamines, les épaississants, les oligo-éléments, les adoucissants, les séquestrants, les parfums, les agents alcalinisants ou acidifiants, les conservateurs, les filtres UV, ou leurs mélanges.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition de l'invention , notamment les compositions de base et de surface, peut être obtenue selon les procédés de préparation classiquement utilisés en cosmétique ou en dermatologie.

Avantageusement, les compositions de base et celle de surface sont conditionnées dans des compartiments ou récipients distincts, pouvant être accompagnés de moyens d'application appropriés, identiques ou différents, tels que des pinceaux, des brosses, des plumes, des éponges, des non tissés (papier, patch).

La composition de surface comprenant les fibres plates peut être appliquée soit à l'une des extrémités de la couche de base, soit au milieu, ou encore de façon discontinue notamment sous forme de motifs géométriques, symétriques ou dissymétriques (par exemple sous forme de points, carrés, ronds, étoiles), répartis de façon aléatoire ou ordonnée, à contours précis ou flous.

Les exemples de compositions ci-après sont donnés à titre illustratif.

**Exemple 1 :**

On a préparé une composition de base de vernis à ongles comprenant :

- nitrocellulose 19 g
- N-éthyl o,p-toluènesulfonamide 6 g
- acétyl citrate de tributyle 6 g
- pigments bleu nuit 1 g
- hectorite 1,2 g
- alcool isopropylique 8 g
- acétate d'éthyle, acétate de butyle qsp 100 g

On a également préparé une composition de surface (vernis à ongles) à milieu translucide comprenant :

- nitrocellulose 17,1 g
- N-éthyl o,p-toluènesulfonamide 5,4 g

- acétyl citrate de tributyle 5,4 g
- Fibres interférentielles de polyéthylène téréphtalate et de nylon de longueur 0,3 mm vendues sous la dénomination "Morphotex" par la société TEIJIN 10 g
- hectorite 1,0 g
- alcool isopropylique 7,2 g
- acétate d'éthyle, acétate de butyle qsp 100 g

Les fibres interférentielles sont bien dispersées de façon homogène dans le milieu solvant du vernis à ongles.
On a appliqué la composition de base de couleur bleu nuit sur les ongles puis après séchage de la couche déposée, on a appliqué sur celle-ci la composition de surface. Après séchage, on obtient un maquillage présentant un dépôt homogène de fibres plates à effet de couleur sur un fond bleu nuit.

**Exemple 2 :**

On a préparé une composition A comprenant :

- nitrocellulose 11,2 g
- résine tosylamide/sulfonamide 11,1 g
- plastifiants 6,8 g
- hectorite 1,1 g
- acétate d'éthyle, acétate de butyle, alcool isopropylique qsp 100 g

On a préparé une composition B comprenant :

- Fibres interférentielles de polyéthylène téréphtalate et de nylon de longueur 0,3 mm vendues sous la dénomination "Morphotex" par la société TEIJIN 10 g
- Composition A 90 g

On a évalué les propriétés viscoélastiques du film obtenu avec les compositions A et B.
Les propriétés viscoélastiques du film sont mesurées lors d'essais dynamiques sous sollicitations sinusoïdales de faible amplitude (petites déformations) réalisés à 30°C sur une plage de fréquence allant de 0,1 à 20 Hz sur un viscoélasticimètre par exemple de type DMA 2980 de T.A. Instruments en traction sur film. Pour cela, chaque mélange est appliqué sur une matrice téflonnée puis après séchage à 30°C pendant 24 heures on récupère le film formé. On mesure ensuite le module élastique en traction E' du film exprimé en MPa.
On a obtenu les résultats suivants :

| Composition | Epaisseur du film (μm) | E' à 0,1 Hz (en MPa) | E' à 1 Hz (en MPa) | E' à 5 Hz (en MPa) | E' à 20 Hz (en MPa) |
|---|---|---|---|---|---|
| **Composition A** | 170 | 6 | 29 | 89 | 203 |
| **Composition B** | 310 | 25 | 76 | 167 | 296 |

On constate que le module élastique mesuré à chaque fréquence du film obtenu avec la composition B comprenant les fibres plates est plus élevé que celui du film obtenu avec la composition A. Ainsi, la présence de fibres plates dans la composition B permet d'obtenir un film présentant des propriétés mécaniques, notamment élastiques, renforcées.

## Revendications

1. Composition cosmétique à application topique à phase continue lipophile, **caractérisée par le fait qu'**elle comprend des fibres plates, dont la section transversale présente une plus grande longueur L1 et une plus petite longueur L2 telles que L1/L2 est supérieur à 4, et dont la section transversale est de forme ovoïdale ou ellipsoïdale, lesdites fibres plates étant des fibres à structure multicouche de polymères comprenant des couches alternées d'au moins un premier polymère et un deuxième polymère, lesdites couches permettant la création d'un effet de couleur par interférences des rayons lumineux, qui diffractent et diffusent différemment

selon les couches.

2. Composition selon la revendication 1, **caractérisée par le fait que** les fibres plates ont une longueur L et un diamètre D tel que UD est choisi dans la gamme allant de 1,2 à 2 500, de préférence de 1,5 à 500, et mieux de 1,6 à 150.

3. Composition selon la revendication 1 ou 2, **caractérisée par le fait que** les fibres ont une section transversale comprise dans un cercle de diamètre allant de 2 nm à 500 μm, de préférence allant de 100 nm à 100 μm et mieux de 1 μm à 70 μm.

4. Composition selon l'une quelconque des revendications précédentes, **caractérisée par le fait que** les fibres plates ont une longueur L allant de 1 μm à 10 mm, de préférence de 0,1 mm à 5 mm et mieux de 0,3 mm à 3,5 mm.

5. Composition selon l'une quelconque des revendications précédentes, **caractérisée par le fait que** les fibres plates ont une section transversale présentant une plus grande longueur L1 et une plus petite longueur L2 telles que L1/L2 est supérieur à 7.

6. Composition selon l'une des revendications 1 à 4, **caractérisée par le fait que** L1/L2 va de 4 à 15, de préférence de 6 à 12, et mieux de 7 à 10.

7. Composition selon l'une quelconque des revendications précédentes, **caractérisée par le fait que** les fibres plates se présentent sous forme de ruban ou de tagliatelle.

8. Composition selon l'une quelconque des revendications précédentes, **caractérisée par le fait que** les fibres sont des fibres monofilament ou multifilaments.

9. Composition selon l'une quelconque des revendications précédentes, **caractérisée par le fait que** les fibres plates sont torsadées le long de l'axe de la longueur L des fibres.

10. Composition selon l'une quelconque des revendications précédentes,
**caractérisée par le fait que** les fibres plates ont un titre choisi dans la gamme allant de 0,15 à 30 deniers et mieux de 0,18 à 18 deniers.

11. Composition selon l'une quelconque des revendications précédentes,
**caractérisée par le fait que** les fibres plates sont des fibres de polymères.

12. Composition selon l'une quelconque des revendications précédentes,
**caractérisée par le fait que** les fibres plates sont choisies parmi les fibres de rayonne, de polyamide, de viscose, d'acétate notamment d'acétate de rayonne, de poly-(p-phénylène-téréphtalamide), de polymère acrylique, notamment de polyméthacrylate de méthyle ou de polyméthacrylate de 2-hydroxyéthyle, de polyoléfine et notamment de polyéthylène ou de polypropylène, de polytétrafluoroéthylène (comme le Téflon®), de polychlorure de vinyle ou de vinylidène, de polyfluorure de vinylidène, d'alcool polyvinylique, de polyacrylonitrile, de polyuréthane, de polyesters comme les polyéthylène téréphtalates, les polyéthylène naphtalates, de polycarbonate.

13. Composition selon l'une quelconque des revendications précédentes,
**caractérisée par le fait que** les fibres plates sont choisies parmi les fibres de polyester, de polymère acrylique, de polyamide.

14. Composition selon l'une quelconque des revendications précédentes,
**caractérisée par le fait que** les fibres plates comprennent un polymère choisi dans le groupe formé par le polyéthylène téréphtalate, le polyéthylène naphtalate, le polycarbonate, le polyméthacrylate de méthyle, le nylon 6, nylon 6-6, nylon 6-12, nylon 11, nylon 12.

15. Composition selon l'une des revendications précédentes, **caractérisée par le fait que** chaque couche de polymère étant dans un plan (P) parallèle à la direction de l'axe principal de la fibre, dans le sens de sa longueur L.

16. Composition selon l'une quelconque des revendications précedentes,
**caractérisée par le fait que** la partie multicouche de la fibre peut comprendre au moins 5 couches individuelles de polymère, notamment de 5 à 120, de préférence au moins 10 couches, notamment de 10 à 70 couches, et mieux

de 10 à 50 couches.

**17.** Composition selon l'une quelconque des revendications précédentes, **caractérisée par le fait que** chaque couche des premier et deuxième polymères a respectivement une épaisseur $d_1$, $d_2$ allant, indépendamment l'une de l'autre, de 0,02 $\mu$m à 0,3 $\mu$m, et de préférence de 0,05 $\mu$m à 0,15 $\mu$m.

**18.** Composition selon l'une quelconque des revendications précédentes, **caractérisée par le fait que** les polymères présents dans les fibres ont un indice de réfraction allant de 1,30 à 1,82 et mieux allant de 1,35 à 1,75.

**19.** Composition selon l'une quelconque des revendications précédentes, **caractérisée par le fait que** les premier et deuxième polymères ont respectivement un indice de réfraction $n_1$ et $n_2$ tels que $n_1/n_2$ va de 1,1 à 1,4.

**20.** Composition selon l'une quelconque des revendications précédentes, **caractérisée par le fait que** la fibre plate à structure multicouche présente un spectre de réflexion tel que la largeur à mi-hauteur du spectre $\lambda_{L=1/2}$ est dans la gamme $0 < \lambda_{L=1/2} < 200$ nm.

**21.** Composition selon l'une quelconque des revendications précédentes, **caractérisée par le fait que** le premier polymère est un polyester et le deuxième polymère est un polyamide.

**22.** Composition selon l'une quelconque des revendications précédentes, **caractérisée par le fait que** les fibres plates sont traitées en surface ou enrobées d'une couche de protection.

**23.** Composition selon la revendication 22, **caractérisée par le fait que** la couche de protection comprend un polymère choisi dans le groupe formé par les polyuréthanes, les polyacrylates d'éthyle, les polyméthacrylates d'éthyle.

**24.** Composition selon la revendication 23, **caractérisée par le fait que** le polymère de la couche de protection a un indice de réfraction allant de 1,35 à 1,55.

**25.** Composition selon l'une quelconque des revendications précédentes, **caractérisée par le fait que** les fibres plates sont présentes en une teneur allant de 0,01 % à 50 % en poids, par rapport au poids total de la composition, de préférence de 0,1 % à 30 % en poids, et mieux de 0,3 % à 20 % en poids.

**26.** Composition selon l'une quelconque des revendications précédentes, **caractérisée par le fait que** la phase continue lipophile contient au moins un corps gras choisi dans le groupe formé par les huiles, les cires, les gommes, les corps gras pâteux, les solvants organiques lipophiles et leurs mélanges.

**27.** Composition selon l'une quelconque des revendications précédentes, **caractérisée par le fait qu'**elle comprend un polymère filmogène.

**28.** Composition selon l'une quelconque des revendications précédentes, **caractérisée par le fait qu'**elle comprend une phase hydrophile, aqueuse, alcoolique ou hydroalcoolique dispersée ou émulsionnée dans la phase continue lipophile.

**29.** Composition selon l'une quelconque des revendications précédentes, **caractérisée par le fait qu'**elle contient, en outre, une phase particulaire présente en une teneur allant de 0 à 48 % du poids total de la composition, de préférence allant de 0,01 % à 30 %, et mieux allant de 0,02 % à 20 %.

**30.** Composition selon la revendication 29, **caractérisée par le fait que** la phase particulaire est choisie dans le groupe formé par les pigments, les nacres et les charges.

**31.** Composition selon l'une des revendications précédentes, **caractérisée par le fait qu'**elle se présente sous forme d'un produit de maquillage de la peau, des lèvres et/ou des phanères d'être humain.

**32.** Composition selon l'une des revendications précédentes, **caractérisée par le fait qu'**elle se présente sous la forme d'un vernis à ongles, de mascara, d'eye-liner, de composition capillaire, de produit pour les lèvres, de fond de teint, de produit anti-cernes, de fard à joues ou à paupières, de produit pour les sourcils, de produit de maquillage du corps.

**33.** Composition selon l'une quelconque des revendications précédentes, **caractérisée par le fait qu'**elle se présente sous la forme de vernis à ongles.

**34.** Procédé cosmétique de maquillage ou de soin des matières kératiniques, **caractérisé par le fait que** l'on applique sur les matières kératiniques une composition selon l'une quelconque des revendications 1 à 33.

**35.** Procédé cosmétique de maquillage des matières kératiniques, **caractérisé par le fait que** l'on applique sur les matières kératiniques :

- une première couche d'une première composition comprenant dans un milieu cosmétiquement acceptable au moins une matière colorante,
- puis à appliquer sur au moins une partie de ladite première couche, une deuxième couche d'une deuxième composition, ladite deuxième composition étant conforme à une composition selon l'une des revendications 1 à 33,

la première composition ne comprenant pas de fibres plates comme présentes dans la deuxième composition.

**36.** Procédé selon la revendication 35, **caractérisé par le fait que** la première et/ou la deuxième composition comprend une phase aqueuse, alcoolique ou hydroalcoolique dispersée ou émulsionnée dans la phase continue lipophile.

**37.** Procédé selon l'une quelconque des revendications 35 ou 36, **caractérisé par le fait que** la première et/ou la deuxième composition contient une phase particulaire présente en une teneur allant de 0 à 48 % du poids total de la composition, de préférence allant de 0,01 % à 30 %, et mieux allant de 0,02 % à 20 %.

**38.** Procédé selon la revendication 37, **caractérisé par le fait que** la phase particulaire est choisie dans le groupe formé par les pigments, les nacres et les charges.

**39.** Procédé selon l'une quelconque des revendications 35 à 38, **caractérisé par le fait que** la deuxième composition comprend un milieu sensiblement translucide.

**40.** Kit de maquillage comprenant:

- une première composition comprenant au moins une matière colorante dans un milieu cosmétiquement acceptable, et
- une deuxième composition, ladite deuxième composition étant conforme à une composition selon l'une des revendications 1 à 33,

la première composition ne comprenant pas de fibres plates comme présent dans la deuxième composition, les première et deuxième compositions étant conditionnées dans des récipients distincts.

**41.** Kit de maquillage selon la revendication 40, **caractérisé par le fait que** fait que la première et/ou la deuxième composition comprend une phase aqueuse, alcoolique ou hydroalcoolique dispersée ou émulsionnée dans la phase continue lipophile.

**42.** Kit de maquillage selon l'une quelconque des revendications 40 ou 41, **caractérisé par le fait que** la première et/ou seconde composition contient une phase particulaire présente en une teneur allant de 0 à 48 % du poids total de la composition, de préférence allant de 0,01 % à 30 %, et mieux allant de 0,02 % à 20 %.

**43.** Kit de maquillage selon la revendication 42, **caractérisé par le fait que** la phase particulaire est choisie dans le groupe formé par les pigments, les nacres et les charges.

**44.** Kit de maquillage selon l'une quelconque des revendications 40 à 43, **caractérisé par le fait que** la première et la deuxième compositions se présentent sous la forme d'un vernis à ongles, de mascara, d'eye-liner, de composition capillaire, de produit pour les lèvres, de fond de teint, de produit pour les sourcils, de produit anti-cernes, de fard à joues ou à paupières, de produit de maquillage du corps.

**45.** Kit de maquillage selon l'une quelconque des revendications 40 à 44, **caractérisé par** le fait la première et la deuxième compositions se présentent sous la forme de vernis à ongles.

**46.** Kit de maquillage selon l'une quelconque des revendications 40 à 45, **caractérisé par le fait que** la deuxième composition comprend un milieu sensiblement translucide.

**47.** Support maquillé comprenant un maquillage susceptible d'être obtenu selon le procédé de maquillage conforme à l'une quelconque des revendications 34 à 39 et appliqué sur ledit support, ledit support étant choisi parmi les faux ongles, les faux cils, les postiches, les perruques, les pastilles ou les patchs adhérents sur la peau ou les lèvres.

**Claims**

**1.** Cosmetic composition for topical application with a lipophilic continuous phase, **characterized in that** it comprises flat fibres, the cross section of which has a longer length L1 and a shorter length L2 such that L1/L2 is greater than 4, and the cross section of which is of ovoid or ellipsoid shape, the said flat fibres being fibres with a multilayer structure of polymers comprising alternate layers of at least a first polymer and a second polymer, the said layers allowing the creation of a colour effect by interferences of light rays, which diffract and diffuse differently according to the layers.

**2.** Composition according to Claim 1, **characterized in that** the flat fibres have a length L and a diameter D such that L/D is chosen from the range going from 1.2 to 2500, preferably from 1.5 to 500, and better still from 1.6 to 150.

**3.** Composition according to Claim 1 or 2,
**characterized in that** the fibres have a cross section included in a circle having a diameter ranging from 2 nm to 500 μm, preferably ranging from 100 nm to 100 μm and better still from 1 μm to 70 μm.

**4.** Composition according to any one of the preceding claims, **characterized in that** the flat fibres have a length L ranging from 1 μm to 10 mm, preferably from 0.1 mm to 5 mm and better still from 0.3 mm to 3.5 mm.

**5.** Composition according to any one of the preceding claims, **characterized in that** the flat fibres have a cross section which has a longer length L1 and a shorter length L2 such that L1/L2 is greater than 7.

**6.** Composition according to one of Claims 1 to 4,
**characterized in that** L1/L2 ranges from 4 to 15, preferably from 6 to 12, and better still from 7 to 10.

**7.** Composition according to any one of the preceding claims, **characterized in that** the flat fibres are provided in the form of ribbons or tagliatelle.

**8.** Composition according to any one of the preceding claims, **characterized in that** the fibres are monofilament or multifilament fibres.

**9.** Composition according to any one of the preceding claims, **characterized in that** the flat fibres are twisted along the axis of the length L of the fibres.

**10.** Composition according to any one of the preceding claims, **characterized in that** the flat fibres have a titre chosen from the range going from 0.15 to 30 denier and better still from 0.18 to 18 denier.

**11.** Composition according to any one of the preceding claims, **characterized in that** the flat fibres are polymer fibres.

**12.** Composition according to any one of the preceding claims, **characterized in that** the flat fibres are chosen from rayon fibre, polyamide fibre, viscose fibre, acetate fibre, in particular rayon acetate fibre, poly(p-phenylene-terephthalamide) fibre, acrylic polymer fibre, in particular polymethyl methacrylate fibre or poly-2-hydroxyethyl methacrylate fibre, polyolefin fibre and in particular polyethylene or polypropylene fibre, polytetrafluoroethylene fibre (such as Teflon®), polyvinyl or polyvinylidene chloride fibre, polyvinylidene fluoride fibre, polyvinyl alcohol fibre, polyacrylonitrile fibre, polyurethane fibre, polyester fibre such as polyethylene terephthalates and polyethylene naphthalates, and polycarbonate fibre.

13. Composition according to any one of the preceding claims, **characterized in that** the flat fibres are chosen from polyester, acrylic polymer and polyamide fibres.

14. Composition according to any one of the preceding claims, **characterized in that** the flat fibres comprise a polymer chosen from the group consisting of polyethylene terephthalate, polyethylene naphthalate, polycarbonate, polymethyl methacrylate, nylon 6, nylon 6-6, nylon 6-12, nylon 11, nylon 12.

15. Composition according to one of the preceding claims, **characterized in that** each layer of polymer is in a plane (P) parallel to the direction of the principal axis of the fibre, in the direction of its length L.

16. Composition according to any one of the preceding claims, **characterized in that** the multilayer part of the fibre may comprise at least 5 individual layers of polymer, in particular from 5 to 120, preferably at least 10 layers, in particular from 10 to 70 layers, and better still from 10 to 50 layers.

17. Composition according to any one of the preceding claims, **characterized in that** each layer of the first and second polymers has respectively a thickness $d_1$, $d_2$ ranging, independently of each other, from 0.02 μm to 0.3 μm, and preferably from 0.05 μm to 0.15 μm.

18. Composition according to any one of the preceding claims, **characterized in that** the polymers present in the fibres have a refractive index ranging from 1.30 to 1.82 and better still ranging from 1.35 to 1.75.

19. Composition according to any one of the preceding claims, **characterized in that** the first and second polymers have respectively a refractive index $n_1$ and $n_2$ such that $n_1/n_2$ ranges from 1.1 to 1.4.

20. Composition according to any one of the preceding claims, **characterized in that** the flat fibre with multilayer structure has a reflection spectrum such that the width at half height of the spectrum $\lambda_{L=1/2}$ is in the range $0 < \lambda_{L=1/2} < 200$ nm.

21. Composition according to any one of the preceding claims, **characterized in that** the first polymer is a polyester and the second polymer is a polyamide.

22. Composition according to any one of the preceding claims, **characterized in that** the flat fibres are surface-treated or coated with a protective layer.

23. Composition according to Claim 22, **characterized in that** the protective layer comprises a polymer chosen from the group consisting of polyurethanes, polyethyl acrylates, polyethyl methacrylates.

24. Composition according to Claim 23, **characterized in that** the polymer of the protective layer has a refractive index ranging from 1.35 to 1.55.

25. Composition according to any one of the preceding claims, **characterized in that** the flat fibres are present in an amount ranging from 0.01% to 50% by weight, relative to the total weight of the composition, preferably from 0.1% to 30% by weight, and better still from 0.3% to 20% by weight.

26. Composition according to any one of the preceding claims, **characterized in that** the lipophilic continuous phase contains at least one fatty substance chosen from the group consisting of oils, waxes, gums, pasty fatty substances, lipophilic organic solvents and mixtures thereof.

27. Composition according to any one of the preceding claims, **characterized in that** it comprises a film-forming polymer.

28. Composition according to any one of the preceding claims, **characterized in that** it comprises an aqueous, alcoholic or aqueous-alcoholic hydrophilic phase dispersed or emulsified in the lipophilic continuous phase.

29. Composition according to any one of the preceding claims, **characterized in that** it contains, in addition, a particulate phase present in an amount ranging from 0 to 48% of the total weight of the composition, preferably ranging from 0.01% to 30%, and better still ranging from 0.02% to 20%.

30. Composition according to Claim 29, **characterized in that** the particulate phase is chosen from the group consisting

of pigments, pearlescent agents and fillers.

**31.** Composition according to one of the preceding claims, **characterized in that** it is provided in the form of a make-up product for the skin, lips and/or superficial body growths of human beings.

**32.** Composition according to one of the preceding claims, **characterized in that** it is provided in the form of a nail varnish, mascara, eyeliner, hair composition, product for the lips, foundation, concealer, blusher or eyeshadow, product for the eyebrows, make-up product for the body.

**33.** Composition according to any one of the preceding claims, **characterized in that** it is provided in the form of a nail varnish.

**34.** Cosmetic method for applying make-up to or caring for keratinous materials, **characterized in that** a composition according to any one of Claims 1 to 33 is applied to the keratinous materials.

**35.** Cosmetic method for applying make-up to keratinous materials, **characterized in that** there is applied to the keratinous materials:

- a first layer of a first composition comprising, in a cosmetically acceptable medium, at least one colouring matter,
- then the application, to at least a portion of the said first layer, of a second layer of a second composition, the said second composition being in accordance with a composition according to one of Claims 1 to 33,

the first composition not comprising flat fibres as present in the second composition.

**36.** Method according to Claim 35, **characterized in that** the first and/or the second composition comprises an aqueous, alcoholic or aqueous-alcoholic phase dispersed or emulsified in the lipophilic continuous phase.

**37.** Method according to either of Claims 35 and 36, **characterized in that** the first and/or the second composition contains a particulate phase present in an amount ranging from 0 to 48% of the total weight of the composition, preferably ranging from 0.01% to 30%, and better still ranging from 0.02% to 20%.

**38.** Method according to Claim 37, **characterized in that** the particulate phase is chosen from the group consisting of pigments, pearlescent agents and fillers.

**39.** Method according to any one of Claims 35 to 38, **characterized in that** the second composition comprises a substantially translucent medium.

**40.** Make-up kit comprising:

- a first composition comprising at least one colouring matter in a cosmetically acceptable medium, and
- a second composition, the said second composition being in accordance with a composition according to one of Claims 1 to 33,

the first composition not comprising flat fibres as present in the second composition,
the first and second compositions being packaged in separate containers.

**41.** Make-up kit according to Claim 40, **characterized in that** the first and/or the second composition comprises an aqueous, alcoholic or aqueous-alcoholic phase dispersed or emulsified in the lipophilic continuous phase.

**42.** Make-up kit according to either of Claims 40 and 41, **characterized in that** the first and/or the second composition contains a particulate phase present in an amount ranging from 0 to 48% of the total weight of the composition, preferably ranging from 0.01% to 30%, and better still ranging from 0.02% to 20%.

**43.** Make-up kit according to Claim 42, **characterized in that** the particulate phase is chosen from the group consisting of pigments, pearlescent agents and fillers.

**44.** Make-up kit according to any one of Claims 40 to 43, **characterized in that** the first and second compositions are provided in the form of a nail varnish, mascara, eyeliner, hair composition, product for the lips, foundation, product

for the eyebrows, concealer, blusher or eyeshadow, make-up product for the body.

**45.** Make-up kit according to any one of Claims 40 to 44, **characterized in that** the first and second compositions are provided in the form of a nail varnish.

**46.** Make-up kit according to any one of Claims 40 to 45, **characterized in that** the second composition comprises a substantially translucent medium.

**47.** Support to which make-up has been applied comprising a make-up which is capable of being obtained according to the make-up application method in accordance with any one of Claims 34 to 39 and applied to the said support, the said support being chosen from false nails, false eyelashes, postiches, wigs, pastilles or patches adhering to the skin or the lips.

## Patentansprüche

**1.** Kosmetische Zusammensetzung für die topische Anwendung mit einer kontinuierlichen lipophilen Phase, **dadurch gekennzeichnet, dass** sie flache Fasern enthält, deren Querschnitt eine größere Länge L1 und eine kleiner Länge L2 aufweist, die so sind, dass L1/L2 größer 4 ist, und deren Querschnitt eiförmig oder ellipsenförmig ist, wobei es sich bei den flachen Fasern um Fasern mit einer Mehrschichtstruktur von Polymeren handelt, die alternierende Schichten mindestens eines ersten Polymers und eines zweiten Polymers aufweist, wobei die Schichten durch Interferenz der Lichtstrahlung, die in Abhängigkeit von den Schichten in unterschiedlicher Weise gebeugt oder gestreut wird, einen Farbeffekts ermöglichen können.

**2.** Zusammensetzung nach Anspruch 1, **dadurch gekennzeichnet, dass** die flachen Fasern einen Länge L und einen Durchmesser D aufweisen, die so sind, dass L/D im Bereich von 1,2 bis 2 500, vorzugsweise 1,5 bis 500 und besser 1,6 bis 150 liegt.

**3.** Zusammensetzung nach Anspruch 1 oder 2, **dadurch gekennzeichnet, dass** die Fasern einen Querschnitt aufweisen, der in einen Kreis mit einem Durchmesser von 2 nm bis 500 μm, vorzugsweise 100 nm bis 100 μm und besser 1 bis 70 μm einbeschrieben werden kann.

**4.** Zusammensetzung nach einem der vorhergehenden Ansprüche, **dadurch gekennzeichnet, dass** die flachen Fasern eine Länge L von 1 μm bis 10 mm, vorzugsweise 0,1 bis 5 mm und besser 0,3 bis 3,5 mm aufweisen.

**5.** Zusammensetzung nach einem der vorhergehenden Ansprüche, **dadurch gekennzeichnet, dass** die flachen Fasern einen Querschnitt mit einer größeren Länge L1 und einer kleinern Länge L2 besitzen, die so sind, dass L1/L2 größer 7 ist.

**6.** Zusammensetzung nach einem der Ansprüche 1 bis 4, **dadurch gekennzeichnet, dass** L1/L2 im Bereich von 4 bis 15, vorzugsweise 6 bis 12 und besser 7 bis 10 liegt.

**7.** Zusammensetzung nach einem der vorhergehenden Ansprüche, **dadurch gekennzeichnet, dass** die flachen Fasern in der Form eines Bandes oder in der Form von Tagliatelle vorliegen.

**8.** Zusammensetzung nach einem der vorhergehenden Ansprüche, **dadurch gekennzeichnet, dass** die Fasern Monofilamente oder Multifilamente sind.

**9.** Zusammensetzung nach einem der vorhergehenden Ansprüche, **dadurch gekennzeichnet, dass** die flachen Fasern entlang der Faserachse der Länge L verdrillt sind.

**10.** Zusammensetzung nach einem der vorhergehenden Ansprüche, **dadurch gekennzeichnet, dass** die flachen Fasern einen Titer aufweisen, der im Bereich von 0,15 bis 30 Denier und besser 0,18 bis 18 Denier ausgewählt ist.

**11.** Zusammensetzung nach einem der vorhergehenden Ansprüche, **dadurch gekennzeichnet, dass** die flachen Fasern Polymerfasern sind.

**12.** Zusammensetzung nach einem der vorhergehenden Ansprüche, **dadurch gekennzeichnet, dass** die flachen Fasern unter den Rayonfasern, Polyamidfasern, Viskosefasern, Acetatfasern und insbesondere Fasern aus Rayonacetat, Poly-(*p*-phenylenterephthalamid)-Fasern, Acrylfasern und insbesondere Fasern aus Polymethylmethacrylat oder Poly-2-hydroxyethylmethacrylat, Polyolefinfasern und insbesondere Fasern aus Polyethylen oder Polypropylen, Polytetrafluorethylenfasern (wie Teflon®), Polyvinylchloridfasern, Polyvinylidenchloridfasern, Polyvinylalkoholfasern, Polyacrylnitrilfasern, Polyurethanfasern, Polyesterfasern, wie Polyethylenterephthalatfasern, Polyethylennaphthalatfasern und Polycarbonatfasern ausgewählt sind.

**13.** Zusammensetzung nach einem der vorhergehenden Ansprüche, **dadurch gekennzeichnet, dass** die flachen Fasern unter Polyesterfasern, Acrylpolymerfasern und Polyamidfasern ausgewählt sind.

**14.** Zusammensetzung nach einem der vorhergehenden Ansprüche, **dadurch gekennzeichnet, dass** die flachen Fasern ein Polymer enthalten, das unter Polyethylenterephthalat, Polyethylennaphthalat, Polycarbonat, Polymethylmethacrylat, Nylon 6, Nylon 6-6, Nylon 6-12, Nylon 11 und Nylon 12 ausgewählt ist.

**15.** Zusammensetzung nach einem der vorhergehenden Ansprüche, **dadurch gekennzeichnet, dass** jede Polymerschicht in einer Ebene (P) parallel zur Richtung der Hauptachse der Faser in der Richtung ihrer Länge L verläuft.

**16.** Zusammensetzung nach einem der vorhergehenden Ansprüche, **dadurch gekennzeichnet, dass** der Multischichtteil der Faser mindestens 5 einzelne Polymerschichten, insbesondere 5 bis 120 Schichten, vorzugsweise mindestens 10 Schichten, insbesondere 10 bis 70 Schichten und besser 10 bis 50 Schichten aufweisen kann.

**17.** Zusammensetzung nach einem der vorhergehenden Ansprüche, **dadurch gekennzeichnet, dass** jede Schicht des ersten bzw. zweiten Polymers eine Dicke $d_1$ bzw. $d_2$ aufweist, die unabhängig voneinander 0,02 bis 0,3 µm und vorzugsweise 0,05 bis 0,15 µm betragen.

**18.** Zusammensetzung nach einem der vorhergehenden Ansprüche, **dadurch gekennzeichnet, dass** die in den Fasern enthaltenen Polymere einen Brechungsindex von 1,30 bis 1,82 und besser 1,35 bis 1,75 aufweisen.

**19.** Zusammensetzung nach einem der vorhergehenden Ansprüche, **dadurch gekennzeichnet, dass** das erste und zweite Polymer einen Brechungsindex $n_1$ bzw. $n_2$ aufweisen, der so ist, dass $n_1/n_2$ im Bereich von 1,1 bis 1,4 liegt.

**20.** Zusammensetzung nach einem der vorhergehenden Ansprüche, **dadurch gekennzeichnet, dass** die flache Faser mit Mehrschichtstruktur ein Reflexionsspektrum aufweist, dass so ist, dass die Halbwertsbreite des Spektrums $\lambda_{L=1/2}$ im Bereich von $0 < \lambda_{L=1/2} < 200$ nm liegt.

**21.** Zusammensetzung nach einem der vorhergehenden Ansprüche, **dadurch gekennzeichnet, dass** das erste Polymer ein Polyester und das zweite Polymer ein Polyamid ist.

**22.** Zusammensetzung nach einem der vorhergehenden Ansprüche, **dadurch gekennzeichnet, dass** die flachen Fasern an der Oberfläche behandelt oder mit einer Schutzschicht überzogen sind.

**23.** Zusammensetzung nach Anspruch 22, **dadurch gekennzeichnet, dass** die Schutzschicht ein Polymer enthält, das unter den Polyurethanen, Polyethylacrylaten und Polyethylmethacrylaten ausgewählt ist.

**24.** Zusammensetzung nach Anspruch 23, **dadurch gekennzeichnet, dass** das Polymer der Schutzschicht einen Brechungsindex von 1,35 bis 1,55 aufweist.

**25.** Zusammensetzung nach einem der vorhergehenden Ansprüche, **dadurch gekennzeichnet, dass** die flachen Fasern in einem Mengenanteil von 0,01 bis 50 Gew.-%, bezogen auf das Gesamtgewicht der Zusammensetzung, vorzugsweise 0,1 bis 30 Gew.-% und besser 0,3 bis 20 Gew.-% enthalten sind.

**26.** Zusammensetzung nach einem der vorhergehenden Ansprüche, **dadurch gekennzeichnet, dass** die kontinuierliche lipophile Phase mindestens eine Fettsubstanz enthält, die unter den Ölen, Wachsen, Gummis, pastösen Fettsubstanzen, lipophilen organischen Lösemitteln und deren Gemischen ausgewählt ist.

**27.** Zusammensetzung nach einem der vorhergehenden Ansprüche, **dadurch gekennzeichnet, dass** sie ein filmbildendes Polymer enthält.

**28.** Zusammensetzung nach einem der vorhergehenden Ansprüche, **dadurch gekennzeichnet, dass** sie eine wässrige, alkoholische oder wässrig-alkoholische hydrophile Phase enthält, die in der kontinuierlichen lipophilen Phase dispergiert oder emulgiert ist.

**29.** Zusammensetzung nach einem der vorhergehenden Ansprüche, **dadurch gekennzeichnet, dass** sie ferner eine Partikelphase enthält, die in einem Mengeanteil von 0 bis 48 % des Gesamtgewichts der Zusammensetzung, vorzugsweise 0,01 bis 30 % und besser 0,02 bis 20 % enthalten ist.

**30.** Zusammensetzung nach Anspruch 29, **dadurch gekennzeichnet, dass** die Partikelphase unter den Pigmenten, Perlglanzpigmenten und Füllstoffen ausgewählt ist.

**31.** Zusammensetzung nach einem der vorhergehenden Ansprüche, **dadurch gekennzeichnet, dass** sie in Form eines Produktes zum Schminken der menschlichen Haut, Lippen und/oder Hautanhangsgebilde ausgewählt ist.

**32.** Zusammensetzung nach einem der vorhergehenden Ansprüche, **dadurch gekennzeichnet, dass** sie als Nagellack, Mascara, Eyeliner, Zusammensetzung für die Haarbehandlung, Produkt für die Lippen, Make-up, Produkt gegen Augenringe, Wangenrouge oder Lidschatten, Produkt für die Augenbrauen oder Produkt zum Schminken des Körpers vorliegt.

**33.** Zusammensetzung nach einem der vorhergehenden Ansprüche, **dadurch gekennzeichnet, dass** sie als Nagellack vorliegt.

**34.** Kosmetisches Verfahren zum Schminken oder für die Pflege von Keratinsubstanzen, **dadurch gekennzeichnet, dass** auf die Keratinsubstanzen eine Zusammensetzung nach einem der Ansprüche 1 bis 33 aufgebracht wird.

**35.** Kosmetisches Verfahren zum Schminken von Keratinsubstanzen, **dadurch gekennzeichnet, dass** auf die Keratinsubstanzen aufgebracht wird:

- eine erste Schicht einer ersten Zusammensetzung, die in einem kosmetisch akzeptablen Medium mindestens ein Farbmittel enthält,
- anschließend wird auf zumindest einem Teil der ersten Schicht eine zweite Schicht aus einer zweiten Zusammensetzung aufgebracht, wobei die zweite Zusammensetzung einer Zusammensetzung nach einem der Ansprüche 1 bis 33 entspricht,
- wobei die erste Zusammensetzung keine flachen Fasern enthält, wie sie in der zweiten Zusammensetzung enthalten sind.

**36.** Verfahren nach Anspruch 35, **dadurch gekennzeichnet, dass** die erste und/ oder zweite Zusammensetzung eine wässrige, alkoholische oder wässrig-alkoholische Phase enthält, die in der kontinuierlichen lipophilen Phase dispergiert oder emulgiert ist.

**37.** Verfahren nach einem der Ansprüche 35 oder 36, **dadurch gekennzeichnet, dass** die erste und/oder zweite Zusammensetzung eine Partikelphase enthält, die in einem Mengenanteil von 0 bis 48 % Gesamtgewichts der Zusammensetzung, vorzugsweise 0,01 bis 30 % und besser 0,02 bis 20 % enthalten ist.

**38.** Verfahren nach Anspruch 37, **dadurch gekennzeichnet, dass** die Partikelphase unter den Pigmenten, Perlglanzpigmenten und Füllstoffen ausgewählt ist.

**39.** Verfahren nach einem der Ansprüche 35 bis 38, **dadurch gekennzeichnet, dass** die zweite Zusammensetzung ein im Wesentlichen durchscheinendes Medium enthält.

**40.** Kit zum Schminken umfassend:

- eine erste Zusammensetzung, die in einem kosmetisch akzeptablen Medium mindestens ein Farbmittel enthält,
- eine zweite Zusammensetzung, wobei die zweite Zusammensetzung einer Zusammensetzung nach einem der Ansprüche 1 bis 33 entspricht,
- wobei die erste Zusammensetzung keine flachen Fasern enthält, wie sie in der zweiten Zusammensetzung enthalten sind,
- wobei die erste und die zweite Zusammensetzung in unterschiedlichen Behältern konfektioniert sind.

**41.** Kit zum Schminken nach Anspruch 40, **dadurch gekennzeichnet, dass** die erste und/oder zweite Zusammensetzung eine wässrige, alkoholische oder wässrig-alkoholische Phase enthält, die in der kontinuierlichen lipophilen Phase dispergiert oder emulgiert ist.

**42.** Kit zum Schminken nach einem der Ansprüche 40 oder 41, **dadurch gekennzeichnet, dass** die erste und/oder zweite Zusammensetzung eine Partikelphase enthält, die in einem Mengeanteil von 0 bis 48 % des Gesamtgewichts der Zusammensetzung, vorzugsweise 0,01 bis 30 % und besser 0,02 bis 20 % enthalten ist.

**43.** Kit zum Schminken nach Anspruch 42, **dadurch gekennzeichnet, dass** die Partikelphase unter den Pigmenten, Perlglanzpigmenten und Füllstoffen ausgewählt ist.

**44.** Kit zum Schminken nach einem der Ansprüche 40 bis 43, **dadurch gekennzeichnet, dass** die erste und die zweite Zusammensetzung als Nagellack, Mascara, Eyeliner, Zusammensetzung für die Haarbehandlung, Produkt für die Lippen, Make-up, Produkt gegen Augenringe, Wangenrouge oder Lidschatten, Produkt für die Augenbrauen oder Produkt zum Schminken des Körpers vorliegen.

**45.** Kit zum Schminken nach einem der Ansprüche 40 bis 44, **dadurch gekennzeichnet, dass** die erste und die zweite Zusammensetzung als Nagellack vorliegen.

**46.** Kit zum Schminken nach einem der Ansprüche 40 bis 45, **dadurch gekennzeichnet, dass** die zweite Zusammensetzung ein im Wesentlichen durchscheinendes Medium enthält.

**47.** Geschminkter Träger, der nach dem Verfahren zum Schminken nach einem der Ansprüche 34 bis 39 erhältlich ist, das auf den Träger angewandt wurde, wobei der Träger unter den falschen Nägeln, künstlichen Wimpern, Perücken, Plaketten und Patches, die auf der Haut oder den Lippen haften, ausgewählt ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- JP 57158714 A
- US 5498407 A
- EP 0106762 A
- FR 1529329 A
- JP 7196440 A
- JP 2000319131 A
- EP 921217 A
- EP 686858 A
- US 5472798 A
- EP 749746 A
- EP 923928 A
- EP 930060 A
- FR 2232303 A
- EP 542669 A
- EP 787730 A
- EP 787731 A
- WO 9608537 A